# EUROPÄISCHE PATENTSCHRIFT

(11) **EP 1 402 914 B1**
(45) Veröffentlichungstag und Bekanntmachung des Hinweises auf die Patenterteilung: **15.08.2007**
(21) Anmeldenummer: 03020717.9
(22) Anmeldetag: 11.09.2003
(51) Int. Cl.: A61M 16/00

(54) **Beatmungsschutz für Mund zu Mund/Nase -beatmung und Verfahren und Vorrichtung zu deren Herstellung**
Breathing protection for mouth to mouth/nose -respiration and method and device for the fabrication thereof
Protection pour respiration bouche à bouche/nez ainsi que méthode et appareil pour sa fabrication

(30) Priorität: 24.09.2002 DE 10244389
(43) Veröffentlichungstag der Anmeldung: 31.03.2004
(73) Patentinhaber: imeco Einwegprodukte GmbH + Co., 63768 Hösbach (DE)
(72) Erfinder: Holtmann, Michael, 63768 Hösbach (DE)
(74) Vertreter: Körfer, Thomas

(56) Entgegenhaltungen:
- WO-A-00/48481
- WO-A-91/16936
- DE-U- 8 801 386
- DE-U- 8 907 096
- US-A- 5 765 551

## Beschreibung

Die Erfindung betrifft eine Beatmungshilfe sowie ein Verfahren und eine Vorrichtung zu deren Herstellung.

Beatmungshilfen bzw. -masken für die Mund-zu-Mund- oder Mund-zu-Nase-Notfallbeatmung sind als solche in unterschiedlichen Ausprägungen bekannt und im Einsatz. All diesen Ausführungsformen ist gemeinsam, daß sie in zentraler Lage des Maskenkörpers, die der Position des Mund-Nase-Bereiches des zu beatmenden Patienten entspricht, eine Öffnung besitzen, durch die die Beatmung des Verletzten durch den beatmenden Nothelfer erfolgt. Um einen direkten Kontakt zwischen Helfer und Patient über diese Öffnung aus hygienischen Gründen und wegen möglicher Infektionsgefahr zu vermeiden, ist die Öffnung mit einem luftdurchlässigen Filtermaterial abgedeckt.

Bei den Beatmungshilfen bzw. -masken handelt es sich entweder um zweidimensionale Beatmungstücher, die die dreidimensionale Gesichtskontur des zu beatmenden Patienten nicht exakt abdecken, oder um dreidimensionale Masken, wie z.B. in der WO 96/05880 dargestellt, die über das Gesicht gestülpt die Gesichtskontur des zu beatmenden Patienten recht gut abdecken. Nachteilig an der zuletzt genannten Beatmungshilfe ist aber die fehlende Faltbarkeit, die zu einem hohen Verpackungs- und Lagerhaltungsvolumen der dreidimensionalen Maske führt.

Der vorliegenden Erfindung liegt daher die Aufgabe zugrunde, eine Beatmungshilfe zu schaffen, die einerseits eine möglichst exakte dreidimensionale Abdeckung des Gesichtes des zu beatmenden Patienten realisiert und andererseits ein möglichst geringes Verpackungs- und Lagerhaltungsvolumen verursacht. Außerdem besteht die Aufgabe darin, ein Verfahren und eine Vorrichtung zur Herstellung der erfindungsgemäßen Beatmungshilfe anzugeben, so daß die Beatmungshilfe möglichst kostengünstig herstellbar ist.

Die Aufgabe wird hinsichtlich der Beatmungshilfe durch die Merkmale des Anspruchs 1 und hinsichtlich des Verfahrens bzw. der Vorrichtung zur Herstellung der Beatmungshilfe durch Anspruch 6 bzw. 17 gelöst.

Die scheinbar konträren Anforderungen der Dreidimensionalität und des geringen Verpackungs- und Stauvolumens der Beatmungshilfe werden in Abgrenzung zum Stand der Technik, bei dem ein einziges Folienstück Verwendung findet, durch eine Kombination zweier Teilfolien erfüllt, die über eine Faltnaht miteinander verbunden sind. Im zusammengefalteten Zustand der Teilfolien realisiert man ein geringes Verpackungs- und Lagerhaltungsvolumen. Im ausgefalteten Zustand bewirkt man eine exakte dreidimensionale Abdeckung des Gesichts des zu beatmenden Patienten. Der präzise Sitz der Beatmungshilfe auf dem Gesicht des zu beatmenden Patienten wird durch eine Konturausprägung der Faltnaht entsprechend dem Stirn-Nase-Mund-Kinn-Profil des Menschen verstärkt.

Eine kostengünstige Herstellung, wie sie bei Einwegartikeln erforderlich ist, ist durch die Beschränkung des Verfahrens auf drei Trenn-, zwei Verbindungs- und einen Faltprozeß gewährleistet. Weitere Kosteneinsparungen können durch die Verwendung von kostengünstigen Materialien wie synthetische Folien und preisgünstigen Filtermaterialien - z.B. Vlies, Filz oder Papier - erzielt werden.

Die Erfindung wird nachstehend unter Bezugnahme auf die Zeichnung anhand von Ausführungsbeispielen näher beschrieben. Es zeigen:
- Fig. 1: eine Prinzipskizze der erfindungsgemäßen Beatmungshilfe;
- Fig. 2: eine Prinzipskizze der Ausgangsfolie mit Verdeutlichung der Linien der einzelnen Bearbeitungsschritte; und
- Fig. 3: eine Prinzipskizze einer erfindungsgemäßen Vorrichtung zur Herstellung der erfindungsgemäßen Beatmungshilfe.

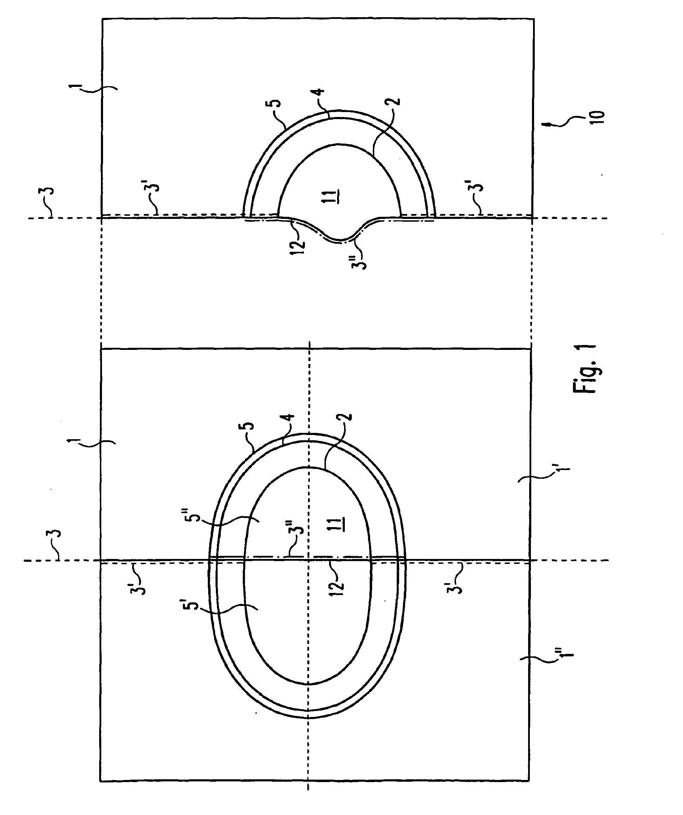

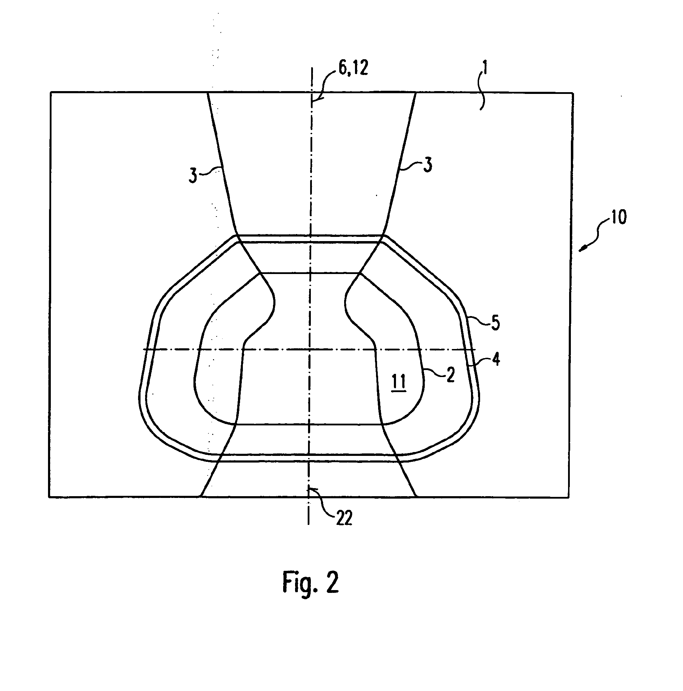

Die erfindungsgemäße Vorrichtung der Beatmungshilfe wird nachfolgend unter Bezugnahme auf Fig. 1 und Fig. 2 beschrieben.

In Fig. 1 ist die Draufsicht auf ein Ausführungsbeispiel der Beatmungshilfe 10 dargestellt. Die Beatmungshilfe 10 besteht aus einer rechteckigen Folie 1, deren Grundzuschnitt in der Höhe dem Scheitel-Kinn-Abstand eines Durchschnittsmenschen und in der Breite dem halben Kopfumfang eines Durchschnittsmenschen auf Ohrenhöhe entspricht. Die Folie 1 besteht aus einem synthetischen Folienmaterial, das sich entsprechend seiner Elastizität optimal der Gesichtsoberfläche des zu beatmenden Patienten anpaßt. Um für den Notfallhelfer einen Augenkontakt zum Patienten zu ermöglichen, ist eine transparente Folie vorteilhaft. Die Folie 1 besitzt in zentraler Lage eine Öffnung 2, die beim Aufsetzen der Beatmungshilfe 10 im Mund-Nase-Bereich positioniert ist und eine runde bis ovale Flächenform besitzt, die der Größe des Mund-Nase-Bereiches eines Durchschnittsmenschen entspricht. Die Folie 1 besteht aus den beiden Teilfolien 1` und 1``, die an den jeweils gegenüberliegenden Seitenrändern einen Profilrand 3' besitzen, der außerhalb der Öffnung 2 dem Stirn-Nase-Mund-Kinn-Konturverlauf eines Durchschnittsmenschen entspricht. Entlang dieses Profilrandes 3` sind die beiden Teilfolien 1` und 1`` über eine feste Verbindung zusammengefügt und bilden eine Folie 1 mit der gemeinsamen Profillinie 3'.

Die Öffnung 2 der Folienschicht 1 ist mit einer Schicht aus luftdurchlässigem Filtermaterial 5 abgedeckt. Hierzu kann jedes Filtermedium wie Vliesstoff, Filz, Papier, Membranmaterialien, Gewebe usw. verwendet werden. Die Schicht aus luftdurchlässigem Filtermaterial 5 besitzt ähnlich wie die Öffnung 2 der Folie 1 eine runde bis ovale Flächenform, weist aber gegenüber der Fläche der Öffnung 2 ein Übermaß auf, um eine vollständige Abdeckung der Öffnung 2 durch die Filtermaterial-Schicht zu gewährleisten. Die Schicht aus luftdurchlässigem Filtermaterial 5 besteht analog zu der Folie aus den beiden Teilabschnitten 5' und 5", die an den jeweils gegenüberliegenden Seitenrändern einer Profillinie 3`` besitzen, der dem Nase-Mund-Konturverlauf eines Durchschnittsmenschen entspricht. Entlang dieser Profillinie 3" sind die beiden Teilabschnitte 5' und 5`` über eine feste Verbindung zusammengefügt und bilden die Filterschicht mit der gemeinsamen Profillinie 3" .

Die Abdeckung 11 aus luftdurchlässigem Filtermaterial 5 steht mit der Folie 1 entlang der gesamten Umrandung 4, die in einem gewissen Abstand zur Umrandung der Öffnung 2 verläuft, in fester Verbindung. Die Verbindung zwischen Abdeckung 11 und Folie 1 ist derart orientiert, daß die Profillinie 3' der Folie 1 und die Profillinie 3`` der Abdeckung 11 aus luftdurchlässigem Filtermaterial 5 die gemeinsame Profillinie 3 ergeben. Somit ergänzen sich die beiden Profillinien 3' (gestrichelt) und 3" (strichpunktiert) gegenseitig in der Ausbildung des vollständigen Stirn-Nase-Mund-Kinn-Verlaufes.

Die erfindungsspezifische Funktionsweise der Beatmungshilfe 10 ergibt sich aus der Verwendung der Profillinie 3 als Faltachse 12. Im zusammengefalteten Zustand liegen die beiden Teilfolien 1` und 1`` sowie die beiden Teilabschnitte 5' und 5`` aufeinander. Die Beatmungshilfe 10 nimmt in diesem Zustand ihr minimales Volumen ein und kann in dieser Stellung platzsparend verpackt und gelagert werden. Um in den ausgeklappten Zustand zu gelangen, werden die beiden Teilfolien 1` und 1`` sowie die beiden Teilabschnitte 5` und 5`` um die als Faltachse 12 dienende Profillinie 3 ausgefaltet. In diesem Zustand weist die Beatmungshilfe 10 ihr maximales dreidimensionales Volumen auf und kann sich somit optimal der dreidimensionalen Kontur der gesichtsseitigen Kopfhälfte des zu beatmenden Patienten anpassen.

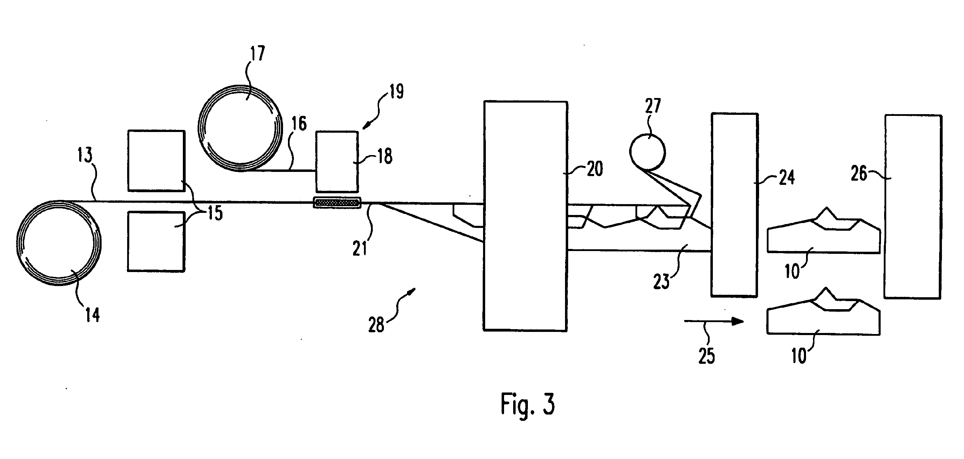

Das erfindungsgemäße Verfahren bzw. die erfindungsgemäße Vorrichtung 28 zur Herstellung der Beatmungshilfe 10 wird nachfolgend unter Bezugnahme auf Fig. 2 und Fig. 3 beschrieben.

Entsprechend Fig. 3 wird die synthetische Folie 1 als Folienbahn 13 von einer Rolle 14 abgewickelt und der ersten Trenneinrichtung 15 zugeführt. In der ersten Trenneinrichtung 15 wird die Öffnung 2 z.B. entsprechend der in Fig. 2 dargestellten Position, Form und Größe aus der Folienbahn 13 getrennt. Als Trennverfahren wird vorzugsweise ein Stanzverfahren eingesetzt. Es kann aber auch jedes andere für synthetisches Folienmaterial geeignete Trennverfahren angewendet werden.

Parallel zur Abwicklung der Folienbahn 13 von der Rolle 14 und zum Trennvorgang der Öffnung 2 aus der Folie 1 wird das luftdurchlässige Filtermaterial 5 ebenfalls als Bahn 16 von einer weiteren Rolle 17, die räumlich hinter der ersten Trenneinrichtung 15 angeordnet ist, abgewickelt. Die Filtermaterialbahn 16 wird entweder quer oder parallel zur Laufrichtung der Filtermaterialbahn 16 einer zweiten Trenneinrichtung 18 zugeführt, in der die Abdeckung 11 z.B. entsprechend der in Fig. 2 dargestellten Form und Größe aus der Filtermaterialbahn 16 getrennt wird. Als Trennverfahren kann analog zur ersten Trenneinrichtung 15 üblicherweise ein Stanzverfahren oder ein anderes für das Filtermaterial 5 geeignetes Trennverfahren zum Einsatz kommen.

Nach dem Trennen der Abdeckung 11 von der Filtermaterialbahn 16 wird in der gleichen Verarbeitungseinheit 19 die Abdeckung 11 über der Öffnung 2 der Folie positioniert. Sobald durch diese Positionierung die Abdeckung 11 aus Filtermaterial 5 mit der Öffnung 2 der Folie 1 entsprechend der Darstellung in Fig. 2 in Deckung kommt, erfolgt eine Verbindung der Abdeckung 11 mit der Folie 1 entlang der gesamten Umrandung 4 der Folienöffnung 2. Als Verbindungsverfahren kann vorzugsweise Schweißen, Ultraschall-Versiegeln oder Verkleben aber auch jedes sonstige für diese beiden Materialien geeignete Befestigungsverfahren herangezogen werden.

Alternativ zu dieser Verfahrensreihenfolge ist auch ein Vertauschen der Verfahrensschritte derart möglich, daß zuerst die Filtermaterialbahn 16 mit der Folienbahn 13 entlang der gesamten Umrandung 4 der Folienöffnung 2 verbunden wird und anschließend in einem zweiten Schritt die Abdeckung 11 entsprechend der in Fig. 2 dargestellten Form und Größe aus der verbundenen Folie-Filtermaterial-Bahn getrennt wird.

Die auf diese Weise hergestellte Bahn des Folie-Filtermaterial-Verbundes 21 wird anschließend einer weiteren Verarbeitungseinheit 20 zugeführt. In dieser Verarbeitungseinheit 20 wird der Folie-Filtermaterial-Verbund entlang der Faltlinie 6 in Fig. 2, die der in Längsrichtung verlaufenden Symmetrieachse 22 der Folienöffnung 2 entspricht, gefaltet.

Nach der Faltung werden in der gleichen Verarbeitungseinheit 20 die übereinander liegenden Falthälften des Folie-Filtermaterial-Verbundes 21 auf der Seite der Faltachse 12 entlang der in Fig. 2 dargestellten Profillinie 3 geschnitten und verbunden. Für den Profilschnitt kann ganz analog wie in Trenneinrichtung 15 und 18 ein Stanzverfahren in Verbindung mit einem Schweißverfahren oder jedes andere für die verwendeten Materialien geeignete Trennverfahren eingesetzt werden. Der durch den Profilschnitt entstandene Folie-Filtermaterial-Abfall wird in der Verarbeitungseinheit 20 automatisch seitlich abgeführt und z.B. in einem Abfallbehälter 27 deponiert.

Anschließend wird die Bahn des gefalteten Folien-Filtermaterial-Verbundes einer weiteren Verarbeitungseinheit 24 zugeführt. In dieser werden die so produzierten Beatmungshilfen 10 aus der Folie-Filtermaterial-Verbund-Bahn 23 vereinzelt, indem sie quer zur Fertigungsrichtung 25 von der Bahn getrennt werden.

Die vereinzelten Beatmungsmasken werden schließlich zu einer letzten Verarbeitungseinheit 26 transportiert, in der sie auf ein beliebiges Verpackungsformat gefaltet werden.

Anstatt nur einer gemeinsamen Öffnung 2 für Mund und Nase können auch getrennte Öffnungen jeweils für Mund oder Nase oder eine Vielzahl kleiner Öffnungen vorgesehen sein.

## Patentansprüche

1. Beatmungshilfe (10) bestehend aus einer Folie (1) mit zumindest einer in zentraler Lage positionierten und der Mund-zu-Mund- und/oder Mund-zu-Nase-Beatmung eines Patienten durch einen Notfallhelfer dienenden Öffnung (2), in die eine Abdeckung (11) aus einem luftdurchlässigen Filtermaterial (5) eingesetzt und im Randbereich verbunden ist,
**dadurch gekennzeichnet,**
**daß** die Folie (1) aus zwei Teilfolien (1', 1") besteht, die entlang einer Profillinie (3'), die zumindest teilweise dem Stirn-Nase-Mund-Kinn-Verlauf des zu beatmenden Patienten entspricht, miteinander verbunden sind, und
**daß** die Abdeckung (11) aus luftdurchlässigem Material (5) mit zwei Teilabschnitten (5', 5")besteht, die entlang einer Profillinie (3"), die dem Mund-Nase-Profilverlauf des zu beatmenden Patienten entspricht, miteinander verbunden sind.

2. Beatmungshilfe nach Anspruch 1,
**dadurch gekennzeichnet,**
**daß** die Folie (1) aus einem transparenten, synthetischen Folienmaterial besteht.

3. Beatmungshilfe nach Anspruch 1 oder 2,
**dadurch gekennzeichnet,**
**daß** das luftdurchlässige Filtermaterial (5) aus den Materialien Vliesstoff, Filz, Papier, Membranmaterial oder Gewebe besteht.

4. Beatmungshilfe nach einem der Ansprüche 1 bis 3,
**dadurch gekennzeichnet,**
**daß** die luftdurchlässige Abdeckung (11) entlang der gesamten Umrandung (4) der Öffnung (2) der Folie (1) derart mit der Folie (1) verbunden ist, daß sich die Profillinie (3'') der luftdurchlässigen Abdeckung (11) und die Profillinie (3') der Folie (1) zur Vervollständigung des Stirn-Nase-Mund-Kinn-Verlaufes gegenseitig ergänzen.

5. Beatmungshilfe nach einem der Ansprüche 1 bis 4,
**dadurch gekennzeichnet,**
**daß** die Profillinie (3) des Stirn-Nase-Mund-Kinn-Verlaufes als Faltachse (12) dient, wobei im zusammengefalteten Zustand die beiden Teilfolien (1', 1'') zur platzsparenden Aufbewahrung der Beatmungshilfe aufeinander liegen und im aufgefalteten Zustand eine dreidimensionale Abdeckung der gesichtsseitigen Kopfhälfte des Patienten durch die Beatmungshilfe ermöglicht ist.

6. Verfahren zur Herstellung einer Beatmungshilfe mit folgenden Verfahrensschritten:
- Trennen einer Öffnung (2) aus einer Folie (1),
- Trennen einer Abdeckung (11) aus einem luftdurchlässigen Filtermaterial (5) entsprechend der Öffnung (2),
- Verbinden der Abdeckung (11) mit der Folie (1) entlang der Umrandung (4) der Öffnung (2) zur Bildung eines Folie-Filtermaterial-Verbundes (21),
- Falten des Folie-Filtermaterial-Verbundes (21) an einer Faltachse (12) , die entlang der in Längsrichtung verlaufenden Symmetrieachse (22) der Öffnung (2) verläuft, zu zwei übereinander liegenden Falthälften und
- Verbinden der Falthälften des Folie-Filtermaterial-Verbundes (21) entsprechend dem Stirn-Nase-Mund-Kinn-Verlauf eines zu beatmenden Patienten und Abtrennen des überstehenden Materials.

7. Verfahren nach Anspruch 6,
**dadurch gekennzeichnet,**
**daß** vor dem Trennen der Öffnung (2) aus der Folie (1) eine Abwicklung einer Folienbahn (13) von einer Rolle (14) und eine Zuführung der Folienbahn (13) zu einer ersten Trenneinrichtung (15) erfolgt.

8. Verfahren nach Anspruch 6 oder 7,
**dadurch gekennzeichnet,**
**daß** vor dem Trennen der Abdeckung (11) aus dem luftdurchlässigen Filtermaterial (5) eine Abwicklung einer Filtermaterialbahn (16) von einer Rolle (17) und eine Zuführung der Filtermaterialbahn (16) zu einer zweiten Trenneinrichtung (18) erfolgt.

9. Verfahren nach einem der Ansprüche 6 bis 8,
**dadurch gekennzeichnet,**
**daß** das Trennen der Öffnung (2) der Folie (1) durch Stanzen erfolgt.

10. Verfahren nach einem der Ansprüche 6 bis 9,
**dadurch gekennzeichnet,**
**daß** das Trennen der Abdeckung aus luftdurchlässigem Filtermaterial (5) durch Stanzen erfolgt.

11. Verfahren nach einem der Ansprüche 6 bis 10,
**dadurch gekennzeichnet,**
**daß** vor dem Verbinden der Abdeckung (11) mit der Folie (1) die Abdeckung (11) über die Öffnung (2) der Folie (1) positioniert wird.

12. Verfahren nach Anspruch 11,
**dadurch gekennzeichnet,**
**daß** das Verbinden der Abdeckung (11) mit der Folie (1) durch Schweißen, Ultraschall-Versiegeln oder Verkleben erfolgt.

13. Verfahren nach einem der Ansprüche 6 bis 12,
**dadurch gekennzeichnet,**
**daß** das Verbinden der Falthälften des Folie-Filtermaterial-Verbundes (21) durch Schweißen, Ultraschall-Versiegeln oder Verkleben erfolgt.

14. Verfahren nach einem der Ansprüche 6 bis 13,
**dadurch gekennzeichnet,**
**daß** nach dem Verbinden der Falthälften des Folie-Filtermaterial-Verbundes (21) die Folie (1) vereinzelt wird.

15. Verfahren nach einem der Ansprüche 6 bis 14,
**dadurch gekennzeichnet,**
**daß** die Vereinzelung durch Trennung von der Folie (1) quer zur Fertigungsrichtung (25) erfolgt.

16. Verfahren nach einem der Ansprüche 6 bis 15,
**dadurch gekennzeichnet,**
**daß** nach der Vereinzelung eine Faltung der Beatmungshilfe (10) auf eine beliebige Verpackungsgröße erfolgt.

17. Vorrichtung (28) zur Herstellung einer Beatmungshilfe (10) mit:
- einer Trenneinrichtung (15), die eine Öffnung (2) aus einer Folie (1) trennt,
- einer ersten Verarbeitungseinheit (19), die eine Abdeckung (11) aus einem luftdurchlässigen Filtermaterial (5) entsprechend der Öffnung (2) der Folie (1) trennt und sie mit der Folie (1) verbindet, und
- einer zweiten Verarbeitungseinheit (20), die den Folie-Filtermaterial-Verbund (21) faltet, die Falthälften des Folie-Filtermaterial-Verbundes verbindet und überstehendes Material abtrennt.

18. Vorrichtung nach Anspruch 17,
**dadurch gekennzeichnet,**
**daß** für die Bevorratung einer unbearbeiteten Folienbahn (13) und einer unbearbeiteten Filtermaterialbahn (16) jeweils eine Abwicklungs-Rolle (14, 17) vorgesehen ist.

19. Vorrichtung nach Anspruch 18,
**gekennzeichnet durch**
eine Vereinzelungseinheit (24), die den gefalteten Folie-Filtermaterial-Verbund vereinzelt.

20. Vorrichtung nach einem der Ansprüche 17 bis 19,
**dadurch gekennzeichnet,**
**daß** eine Verarbeitungseinheit (26) zum Falten der vollständigen Beatmungshilfe vorgesehen ist.

## Claims

1. A ventilation aid (10) comprising a film (1) having at least one opening (2) which is positioned centrally and serves for mouth-to-mouth and/or mouth-to-nose ventilation of a patient by an emergency helper, and into which a cover (11) comprising an air-permeable filter material (5) is inserted and is connected in the edge region, **characterised in that** the film (1) comprises two film parts (1', 1" ) which are connected to one another along a profile line (3') corresponding at least in part to the curve of the forehead/nose/mouth/chin of the patient to be ventilated, and **in that** the cover (11) comprises air-permeable material (5) having two part portions (5', 5'') which are connected to one another along a profile line (3") corresponding to the curve of the mouth/nose profile of the patient to be ventilated.

2. A ventilation aid according to Claim 1, **characterised in that** the film (1) comprises a transparent synthetic film material.

3. A ventilation aid according to Claim 1 or 2, **characterised in that** the air-permeable filter material (5) is made from the materials fleece, felt, paper, membrane material or fabric.

4. A ventilation aid according to one of Claims 1 to 3, **characterised in that** the air-permeable cover (11) is connected to the film (1) along the entire periphery (4) of the opening (2) of the film (1) such that the profile line (3'') of the air-permeable cover (11) and the profile line (3') of the film (1) provide a continuation of one another in order to complete the curve of the forehead/nose/mouth/ chin.

5. A ventilation aid according to one of Claims 1 to 4, **characterised in that** the profile line (3) of the curve of the forehead/nose/mouth/chin serves as an axis of folding (12), and in the folded-together condition the two film parts (1', 1") lie on one another for space-saving storage of the ventilation aid, and in the unfolded condition three-dimensional covering of that half of the patient's head including the face is made possible by the ventilation aid.

6. A method for making a ventilation aid, having the following method steps:
- cutting an opening (2) in a film (1),
- cutting a cover (11) out of an air-permeable filter material (5) to correspond to the opening (2),
- connecting the cover (11) to the film (1) along the periphery (4) of the opening (2), to form a combined film/filter material article (21),
- folding the combined film/filter material article (21) at an axis of folding (12) which runs along the axis of symmetry (22) of the opening (2), in the longitudinal direction, to give two folded halves lying one on top of the other, and
- connecting the folded halves of the combined film/filter material article (21) to correspond to the curve of the forehead/nose/mouth/chin of a patient to be ventilated, and cutting away the overhanging material.

7. A method according to Claim 6, **characterised in that** before the opening (2) is cut in the film (1), a film web (13) is unwound from a roll (14) and the film web (13) is supplied to a first cutting means (15).

8. A method according to Claim 6 or 7, **characterised in that** before the cover (11) is cut out of the air-permeable filter material (5), a filter material web (16) is unwound from a roll (17) and the filter material web (16) is supplied to a second cutting means (18).

9. A method according to one of Claims 6 to 8, **characterised in that** the opening (2) is cut in the film (1) by punching.

10. A method according to one of Claims 6 to 9, **characterised in that** the cover is cut out of air-permeable filter material (5) by punching.

11. A method according to one of Claims 6 to 10, **characterised in that** before the cover (11) is connected to the film (1) the cover (11) is positioned over the opening (2) in the film (1).

12. A method according to Claim 11, **characterised in that** the cover (11) is connected to the film (1) by welding, ultrasound sealing or adhesion.

13. A method according to one of Claims 6 to 12, **characterised in that** the folded halves of the combined film/filter material article (21) are connected by welding, ultrasound sealing or adhesion.

14. A method according to one of Claims 6 to 13, **characterised in that** after the folded halves of the combined film/filter material article (21) have been connected the film (1) is divided into individual pieces.

15. A method according to one of Claims 6 to 14, **characterised in that** the division into individual pieces is performed by cutting the film (1) in a direction transverse to the direction of manufacture (25).

16. A method according to one of Claims 6 to 15, **characterised in that** after the division into individual pieces the ventilation aid (10) is folded to any desired packing size.

17. A device (28) for making a ventilation aid (10), having:
- a cutting means (15) which cuts an opening (2) in a film (1),
- a first processing unit (19) which cuts a cover (11) out of an air-permeable filter material (5) to correspond to an opening (2) in the film (1) and connects it to the film (1), and
- a second processing unit (20) which folds the combined film/filter material article (21), connects the folded halves of the combined film/filter material article and cuts away overhanging material.

18. A device according to Claim 17, **characterised in that** a respective unwinding roll (14, 17) is provided for storing an unprocessed film web (13) and an unprocessed filter material web (16).

19. A device according to Claim 18, **characterised by** a division unit (24) which divides the folded combined film/filter material article into individual pieces.

20. A device according to one of Claims 17 to 19, **characterised in that** a processing unit (26) for folding the completed ventilation aid is provided.

## Revendications

1. Aide respiratoire (10) constituée d'une feuille (1) comportant au moins une ouverture (2) positionnée au centre et servant à la ventilation bouche à bouche et/ou bouche à nez d'un patient par un secouriste, dans laquelle se trouve un recouvrement (11) constitué d'une matière filtrante (5) perméable à l'air et relié dans une zone marginale,
**caractérisée en ce que**
la feuille (1) est constituée de deux parties de feuille (1', 1 ") qui sont reliées entre elles le long d'une ligne profilée (3') qui correspond au moins en partie au parcours front-nez-bouche-menton du patient à ventiler, et
**en ce que** le recouvrement (11) est constitué d'une matière perméable à l'air (5) comportant deux morceaux (5', 5") qui sont reliés entre eux le long d'une ligne profilée (3") qui correspond au parcours bouche-nez du patient à ventiler.

2. Aide respiratoire selon la revendication 1,
**caractérisée en ce que**,
la feuille (1) est constituée d'une matière synthétique transparente.

3. Aide respiratoire selon la revendication 1 ou la revendication 2,
**caractérisée en ce que**
la matière filtrante perméable à l'air (5) est constituée des matériaux suivants : non-tissé, feutre, papier, matière membranaire ou tissu.

4. Aide respiratoire selon l'une quelconque des revendications 1 à 3,
**caractérisée en ce que**
le recouvrement perméable à l'air (11) est relié à la feuille (1) le long de toute la bordure périphérique (4) de l'ouverture (2) de la feuille (1) de manière à ce que la ligne profilée (3") du recouvrement perméable à l'air (11) et la ligne profilée (3') de la feuille (1) se complètent mutuellement pour compléter le parcours front-nez-bouche-menton.

5. Aide respiratoire selon l'une quelconque des revendications 1 à 4,
**caractérisée en ce que**
la ligne profilée (3) du parcours front-nez-bouche-menton sert d'axe de pliage (12), les deux parties de feuille (1', 1 ") se trouvant l'une sur l'autre en situation repliée pour ranger l'aide respiratoire sans prendre trop de place et un recouvrement tridimensionnel de la moitié de la tête du patient côté visage par l'aide respiratoire étant possible en situation dépliée.

6. Procédé de fabrication d'une aide respiratoire comportant les étapes suivantes:
- Pratiquer une ouverture (2) dans la feuille (1)
- Découper un recouvrement (11) dans une matière filtrante perméable à l'air (5) conformément à l'ouverture (2),
- Relier le recouvrement (11) à la feuille (1) le long de la bordure périphérique (4) de l'ouverture (2) pour former un composite feuille-matière filtrante (21),
- Plier le composite feuille-matière filtrante (21) sur un axe de pliage (12) passant le long de l'axe de symétrie (22) longitudinal de l'ouverture (2), en deux moitiés superposées et
- Relier les moitiés du composite feuille-matière filtrante (21) conformément au parcours front-nez-bouche-menton d'un patient à ventiler et découper la matière qui dépasse.

7. Procédé selon la revendication 6,
**caractérisé en ce qu'**
on déroule une bande de feuille (13) d'un rouleau (14) et on amène la bande de feuille (13) vers un premier dispositif de découpe (15) avant de pratiquer l'ouverture (2) dans la feuille (1).

8. Procédé selon la revendication 6 ou la revendication 7,
**caractérisé en ce qu'**
on déroule une bande de matière filtrante (16) d'un rouleau (17) et on amène la bande de matière filtrante (16) vers un deuxième dispositif de découpe (18) avant de découper le recouvrement (11) dans la matière filtrante perméable à l'air (5).

9. Procédé selon l'une quelconque des revendications 6 à 8,
**caractérisé en ce qu'**
on pratique l'ouverture (2) dans la feuille (1) par découpe à l'emporte-pièce.

10. Procédé selon l'une quelconque des revendications 6 à 9,
**caractérisé en ce qu'**
on découpe le recouvrement dans la matière filtrante perméable à l'air (5) par découpe à l'emporte-pièce.

11. Procédé selon l'une quelconque des revendications 6 à 10,
**caractérisé en ce qu'**
on positionne le recouvrement (11) au-dessus de l'ouverture (2) de la feuille (1) avant de relier le recouvrement (11) à la feuille (1).

12. Procédé selon la revendication 11,
**caractérisé en ce qu'**
on relie le recouvrement (11) à la feuille (1) par soudage, scellement aux ultrasons ou collage.

13. Procédé selon l'une quelconque des revendications 6 à 12,
**caractérisé en ce qu'**
on relie les moitiés du composite feuille-matière filtrante (21) par soudage, scellement aux ultrasons ou collage.

14. Procédé selon l'une quelconque des revendications 6 à 13,
**caractérisé en ce qu'**
on désassocie la feuille (1) après avoir relié les moitiés du composite feuille-matière filtrante (21).

15. Procédé selon l'une quelconque des revendications 6 à 14,
**caractérisé en ce que**
la désassociation est réalisée par séparation de la feuille (1) transversalement au sens de fabrication (25).

16. Procédé selon l'une quelconque des revendications 6 à 15,
**caractérisé en ce qu'**
on plie l'aide respiratoire (10) à une dimension d'emballage quelconque après la désassociation.

17. Dispositif (28) de fabrication d'une aide respiratoire (10) comportant :
- un dispositif de découpe (15) pour pratiquer une ouverture (2) dans une feuille (1),
- une première unité de transformation (19) qui découpe un recouvrement (11) dans une matière filtrante perméable à l'air (5) conformément à l'ouverture (2) de la feuille (1) et la relie à la feuille (1), et
- une deuxième unité de transformation (20) qui plie le composite feuille-matière filtrante (21), relie les moitiés du composite feuille-matière filtrante et découpe la matière qui dépasse.

18. Dispositif selon la revendication 17,
**caractérisé en ce qu'**
on prévoit respectivement un rouleau (14, 17) qui fournit une bande de feuille vierge (13) et une bande de matière filtrante vierge (16).

19. Dispositif selon la revendication 18,
**caractérisé par**
une unité de désassociation (24) qui désassocie le composite feuille-matière filtrante.

20. Dispositif selon l'une quelconque des revendications 17 à 19,
**caractérisé en ce qu'**
on prévoit une unité de transformation (26) pour plier l'aide respiratoire complète.
